# EUROPÄISCHE PATENTSCHRIFT

(11) **EP 4 003 910 B1**
(45) Veröffentlichungstag und Bekanntmachung des Hinweises auf die Patenterteilung: **01.11.2023**
(21) Anmeldenummer: 20739338.0
(22) Anmeldetag: 08.07.2020
(51) Int. Cl.: C01B 3/38, C01C 1/04, C07C 29/151, C07C 273/04

(54) **REFORMERANORDNUNG ZUR HERSTELLUNG VON SYNTHESEGAS UND/ODER WASSERSTOFF**
REFORMER ARRANGEMENT FOR PRODUCTION OF SYNTHESIS GAS AND/OR HYDROGEN
AGENCEMENT DE REFORMEURS POUR LA PRODUCTION DE GAZ DE SYNTHÈSE ET/OU D'HYDROGÈNE

(30) Priorität: 26.07.2019 DE 102019211133
(43) Veröffentlichungstag der Anmeldung: 01.06.2022
(73) Patentinhaber: thyssenkrupp Uhde GmbH, 44141 Dortmund (DE); thyssenkrupp AG, 45143 Essen (DE)
(72) Erfinder: SIGGE, Sebastian, 44575 Castrop-Rauxel (DE); SCHOLZ, Marco, 44287 Dortmund (DE)
(74) Vertreter: thyssenkrupp Intellectual Property GmbH
(86) Internationale Anmeldenummer: PCT/EP2020/069268
(87) Internationale Veröffentlichungsnummer: WO 2021/018536

(56) Entgegenhaltungen:
- EP-A1- 0 033 128
- EP-A1- 2 676 924
- EP-A2- 0 382 442
- US-A1- 2010 022 668

## Beschreibung

Die Erfindung betrifft eine Reformeranordnung zur Herstellung von Synthesegas und/oder Wasserstoff, ein Verfahren zur Reformierung eines Gasgemisches aus Dampf und Kohlenwasserstoffen und eine Anlage zur Herstellung von Wasserstoff, Ammoniak, Synthesegas, Harnstoff und/oder Methanol.

Die Erzeugung von Wasserstoff, insbesondere zur Herstellung von Ammoniak und Methanol ist ein wichtiger Basisprozess in der chemischen Industrie.

Ammoniak ist die weltweit zweitmeist produzierte synthetische Chemikalie (Ullmannn's Encyclopedia of Industrial Chemistry, 2012, Wiley-VCH Verlag GmbH &Co. KGaA, Weinheim, DOI:10.1002/14356007.o02_o11, im folgenden "Ullmann's").

Die Herstellung von Ammoniak erfolgt dabei im Wesentlichen aus den Elementen Wasserstoff und Stickstoff in Anwesenheit eines Eisenkatalysators. Die Temperaturen bewegen sich häufig im Bereich zwischen 400 °C und 500 °C und bei einem Druck über 100 bar. Der wesentliche Faktor für die Prozesskosten liegen dabei in der Bereitstellung von Wasserstoff aus der Synthesegas Herstellung (Ullmann's, Seite 139).

Eine Erzeugung von Ammoniak erfolgt dementsprechend bevorzugt im Grundsatz wie beispielsweise bei Holleman, Wiberg, Lehrbuch der Anorganischen Chemie, 102 Auflage, 2007, Seiten 662-665 (ISBN 978-3-11-017770-1) beschrieben, basierend auf dem "Haber-Bosch-Verfahren" aus den Elementen gemäß Gleichung [1]:

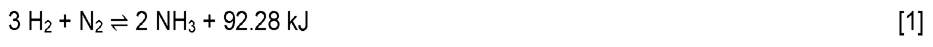

Das Edukt Stickstoff (N₂) kann beispielsweise durch Tieftemperaturluftzerlegung gewonnen werden. Der Wasserstoff wird bevorzugt über den "Steam-Reforming-Prozess" (z.B. wie in Andreas Jess, Peter Wasserscheid, Chemical Technology, An Integrated Textbook, Wiley-VCH, 2013, Seite 536 bis 539, ISBN 978-3-527-30446-2 beschrieben) gemäß Gleichung [2] erhalten:

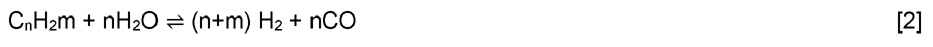

In der anschließenden "Kohlendioxid-Konvertierung" erfolgt eine weitere Umsetzung gemäß Gleichung (3):

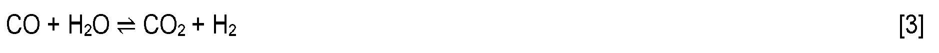

Auch Methanol ist mit einer Jahresproduktion 2007 von 52,1 × 10⁶ t (Saade, G.A. (2009) Chemical Economics Handbook-SRI Consulting) eine bedeutende Grundchemikalie. Wie in "Andreas Jess, Peter Wasserscheid, Chemical Technology, An Integrated Textbook, Wiley-VCH, 2013, Seite 686 bis 694, ISBN 978-3-527-30446-2" beschrieben, lässt sich die Umsetzung vereinfacht über die Gleichungen [4] und [5] beschreiben:

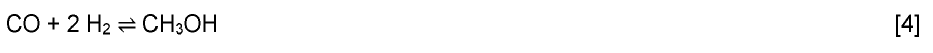

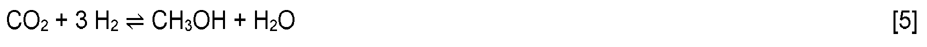

Geeignete Reaktionsbedingungen und Katalysatoren für die Methanolsynthese lassen sich ebenfalls der obigen Literaturstelle entnehmen.

Von großer Bedeutung ist die Verwendung des Wasserstoffs auch in Raffinerien. Der Wasserstoff wird dabei vor allem im Rahmen der Entschwefelung der Rohölprodukte eingesetzt.

Im Zuge stetiger Entwicklungsarbeiten und Verbesserungen von Reformern zu Wasserstoffherstellung wurde der gasbeheizte Reformer (Gas Heated Reformer/GHR) entwickelt. Mit Hilfe eines gasbeheizten Reformers nach dem Oberbegriff des Anspruchs 1, wie beispielsweise in der US 2010/0022668 A1, z.B. Paragraph [0005], [0014] oder [0020] beschrieben, sind eine Kapazitätsvergrößerung in der Reformierung und eine Verbesserung der Wärmebilanz möglich. In einem gasbeheizten Reformer wird ein Teil des vorher abgetrennten Edukt Gas/Dampf Gemisches, beispielsweise Erdgas/Wasserdampf, zu Synthesegas umgesetzt. Dazu wird die für die endotherme Umsetzung erforderliche Wärme beispielsweise durch Nutzung der Wärme des im Primärreformer erzeugten Synthesegases bereitgestellt.

Das ursprüngliche Design des gasbeheizten Reformers (GHR) weist jedoch häufig konstruktive Nachteile auf: Durch den Kontakt des heißen Prozessgases mit der enthaltenden Rohrplatte, kann nur eine begrenzte Anzahl von Reformerrohren installiert werden. Gleichzeitig ist die Einsatzgas/Dampf Vorwärmtemperatur häufig auf unter 500 °C beschränkt und eine teure Ausmauerung der kompletten Gaseinlasskammer ist erforderlich.

US 2004/0018144 A1 offenbart ein Verfahren zur Herstellung von Synthesegas. Der apparative Aufbau umfasst einen Autothermen Reformer (ATR) und einen gasbeheizten Reformer (GHR).

US 4,910,228 A offenbart einen Prozess zur Herstellung von Methanol.

US 2010/0022668 A1 offenbart ein System und Verfahren zur Verarbeitung von Wasserstoff und Kohlenmonoxid. Der offenbarte apparative Aufbau umfasst einen Autothermen Reformer (ATR) und einen gasbeheizten Reformer (GHR).

EP 1 464 910 A2 offenbart einen Apparat und ein Verfahren zur Vermeidung von Metall Korrosion in einem Rohrbündelwärmetauscher.

US 9,126,172 B2 offenbart Reformer für die Herstellung von Synthesegas mit integrierter Vorrichtung für die Shift-Reaktion.

Aus EP 0 033 128 A1 und EP 0 382 442 A2 sind weitere Vorrichtungen zur Dampfreformierung eines kohlenwasserstoffhaltigen Einsatzstoffstroms bekannt, die einen Hauptreformer und einen zweiten, gasbeheizten Reformer aufweisen. In beiden Vorrichtungen erfolgt die Abführung des Produktgasstroms in Höhe des oberen Endes der Reformerrohre des gasbeheizten Reformers.

Die Erfindung hat die Aufgabe die voranstehend genannten Nachteile des Standes der Technik zu vermeiden. Insbesondere die teure, wärmeisolierende Ausmauerung zwischen dem gasbeheizten Reformer und dem Wärmeüberträger (beispielsweise Prozessgaskühler und/oder Prozesskondensatverdampfer) sollte auf ein notwendiges Minimum beschränkt sein.

Die Aufgabe wird überraschenderweise durch eine Reformeranordnung zur Herstellung von Synthesegas und/oder Wasserstoff gemäß Anspruch 1 gelöst.

Vorteilhafte Ausgestaltungen gehen aus den Unteransprüchen hervor.

Die Aufgabe wird des Weiteren durch ein Verfahren zur Reformierung eines Gasgemisches aus Dampf und Kohlenwasserstoffen nach Anspruch 7 gelöst.

Vorteilhafte Ausgestaltungen des Verfahrens gehen aus den Unteransprüchen hervor.

Weiterhin sind zur Lösung der Aufgabe die Verwendung einer erfindungsgemäßen Reformeranordnung zur Herstellung von Wasserstoff, Ammoniak, Synthesegas, Harnstoff und/oder Methanol mit umfasst.

Die Erfindung umfasst des Weiteren eine Anlage zur Herstellung von Wasserstoff, Ammoniak, Synthesegas, Harnstoff und/oder Methanol umfassend eine erfindungsgemäße Reformeranordnung.

Die erfindungsgemäße Reformeranordnung zur Herstellung von Synthesegas und/oder Wasserstoff umfasst die folgenden Elemente. Ein Hauptreformer, beispielsweise ein Primärreformer, ist mit einer Hauptreformer-Edukt-Zuleitung und einer Hauptreformer-Produkt-Ableitung verbunden. Eine Beschreibung der Funktionsweise von Primärreformer, Sekundärreformer, gasbeheizte Reformer (GHR) und Autothermen Reformer (ATR) findet sich beispielsweise in *"*Max Appl, Ullmanns Encyclopedia of Industrial Chemistry, Ammonia, 2. Production Processes, Wiley-VCH Verlag GmbH & Co. KGaA, Weinheim, 2012, Seiten 174-178, DOI: 10.1002/14356007.o02_o11." Weiterhin ist ein gasbeheizter Reformer (GHR/Gas Heated Reformer) umfasst. Der gasbeheizte Reformer (GHR) beinhaltet einen GHR-Reformerinnenraum mit darin angeordneten GHR-Reformerrohren, einen GHR-Rohgaseinlass verbunden mit den GHR-Reformerrohren. Der Ausdruck "GHR-Rohgaseinlass verbunden mit den GHR-Reformerrohren" umfasst im Sinne der Erfindung auch einen Zwischenraum, welcher einen Transport eines Dampf/Kohlenwasserstoff (z.B. Erdgas)-Gemischs von dem GHR-Rohgaseinlass zu den GHR-Reformerrohren ermöglicht. Weiterhin ist eine GHR-Ummantelung der GHR-Reformerrohre vorgesehen. Ein GHR-Hauptreformerproduktgaseinlass ist sowohl mit der Hauptreformer-Produkt-Ableitung und der GHR-Ummantelung verbunden. Ein GHR-Produktauslass ermöglicht einen Austritt des im gasbeheizten Reformer (GHR) umgesetzten Gasgemisches. Die Reformeranordnung umfasst weiterhin einen Wärmeüberträger mit einer Wärmeüberträgereinlassöffnung und einer Wärmeüberträgerauslassöffnung, wobei ein Wärmekanal zwischen der Wärmeüberträgereinlassöffnung und dem GHR-Produktauslass angeordnet ist. Beispiele für geeignete Wärmeüberträger sind Prozessgaskühler und/oder Prozesskondensatverdampfer und/oder Dampfüberhitzer. Die erfindungsgemäße Reformeranordnung ist dadurch gekennzeichnet, dass der GHR-Produktauslass auf einer parallelen Ebene mit dem Wärmeüberträger angeordnet ist und sich die Höhe gemessen vom Boden bis zur Mittelachse des GHR-Produktauslass um maximal plus/minus 20 % von der Höhe gemessen vom Boden bis zum zur Mittelachse der Wärmeüberträgereinlassöffnung unterscheidet. Aufgrund dieser erfindungsgemäßen parallelen Anordnung muss nur eine minimale Verbindungsstrecke als Wärmekanal zwischen Hauptreformer, beispielsweise ein Primärreformer oder ATR, wärmeisoliert werden. Zudem vermeidet die direkte Verbindung Eckstücke, welche andererseits zur Überbrückung von Höhenunterschieden notwendig wären. Insbesondere im Bereich von Eckstücken und Krümmungen des Wärmekanals ist eine Wärmeisolierung, beispielswiese als feuerfeste Ausmauerung, schwierig und teuer zu realisieren. Die Ausdrücke "verbunden", "verbinden" bzw. "Verbindung" umfasst im Sinne der Erfindung geeignete Verbindungsstücke, Rohre, Verbindungsräume, Kompressoren welche einen Übertritt eines Gasgemisches von einem Bauteil in ein anderes Bauteil ermöglichen. Auch können weitere Bauteile, welche einen Transport/Durchtritt eines Gasgemisches erlauben, im Sinne der Erfindung mit umfasst sein.

In einer bevorzugten Ausgestaltung umfasst der Hauptreformer Primärreformer, Sekundärreformer, POX-Reaktor (Partielle Oxidation) und/oder ATR-Reformer. Mit umfasst sind dabei auch ein einzelner Primärreformer oder ATR-Reformer, genauso wie Kombinationen von Primärreformer und Sekundärreformer bzw. Primärreformer und ATR-Reformer.

Bevorzugt umfasst der Wärmeüberträger Prozessgaskühler und/oder Prozesskondensatverdampfer und/oder Dampfüberhitzer.

Die Auslässe der GHR-Reformerrohre sind innerhalb der GHR-Ummantelung angeordnet. Die genannte Anordnung ermöglicht eine Durchmischung des durch die GHR-Reformerrohre reformierten Dampf/Kohlenwasserstoff (z.B. Erdgas)-Gemischs mit dem im Hauptreformer reformierten Dampf/Kohlenwasserstoff(z.B. Erdgas)-Gemischs innerhalb der GHR-Ummantelung.

Bevorzugt ist der GHR-Produktauslass direkt in kürzester, gerader Strecke über den Wärmekanal mit dem Wärmeüberträger verbunden. Diese Anordnung verringert weiterhin den Isolierungsbedarf des Wärmekanals.

In einer weiteren bevorzugten Ausgestaltung, insbesondere in einer Anlage zur Ammoniakherstellung ist zwischen der Hauptreformer-Produkt-Ableitung und dem GHR-Hauptreformerproduktgaseinlass ein Sekundärreformer angeordnet.

Bevorzugt ist der Wärmekanal von einer Wärmekanalisolierung, beispielsweise Steine, Mauersteine, Schamott, Mineralwolle, umgeben.

Die Erfindung umfasst des Weiteren ein Verfahren zur Reformierung eines Gasgemisches aus Dampf und Kohlenwasserstoffen mindestens umfassend die folgenden Schritte. In einem ersten Schritt erfolgt ein Bereitstellen eines Gasgemisches aus Wasserdampf und einen gasförmigen Kohlenwasserstoff, insbesondere Erdgas, Naphtha, LPG (Liquefied Petroleum Gas), kohlenwasserstoffhaltige Raffineriegase, sowie vorreformiertes Gas (Einsatzstoff nach einem Pre-Reformer). In Prozessrichtung schließt sich ein einleiten und Umsetzen von 70 Vol. % bis 90 Vol. % des Gasgemisches als Hauptstrom in einen Hauptreformer und einleiten von 30 Vol. % bis 10 Vol. % des Gasgemisches als Nebenstrom (8b) in einen gasbeheizten Reformer (GHR/Gas Heated Reformer) an. Der gasbeheizte Reformer (GHR) umfasst einen GHR-Produktauslass, wobei der GHR-Produktauslass auf einer parallelen Ebene mit einem Wärmeüberträger (5) angeordnet ist und sich die Höhe gemessen vom Boden bis zur Mittelachse des GHR-Produktauslass (4e) um maximal plus/minus 20 % von der Höhe gemessen vom Boden bis zum zur Mittelachse der Wärmeüberträgereinlassöffnung unterscheidet. In einem weiteren Schritt erfolgt ein Einleiten des umgesetzten Hauptstroms aus dem Hauptreformer in den GHR-Hauptreformerproduktgaseinlass. Gleichzeitig erfolgt ein Erwärmen des Nebenstroms in dem GHR-Reformerinnenraum über den umgesetzten Hauptstrom und erhalten eines gemischten Stroms aus umgesetzten Hauptstrom und umgesetzten Nebenstrom. Daran schließt sich ein Einleiten des gemischten Stroms in den Wärmeüberträger über einen Wärmekanal zwischen der Wärmeüberträgereinlassöffnung und dem GHR-Produktauslass an. Aufgrund dieser erfindungsgemäßen parallelen Anordnung muss nur eine minimale Verbindungsstrecke als Wärmekanal zwischen Hauptreformer, beispielsweise ein Primärreformer oder ATR, wärmeisoliert werden. Zudem vermeidet die direkte Verbindung Eckstücke, welche andererseits zur Überbrückung von Höhenunterschieden notwendig wären. Insbesondere im Bereich von Eckstücken und Krümmungen des Wärmekanals ist eine Wärmeisolierung, beispielswiese als feuerfeste Ausmauerung, schwierig und teuer zu realisieren.

Bevorzugt umfasst der Hauptreformer Primärreformer, Sekundärreformer, POX-Reaktor (Partielle Oxidation) und/oder ATR-Reformer.

In der erfindungsgemäßen Ausgestaltung des Verfahrens umfasst der gasbeheizte Reformer (GHR/Gas Heated Reformer) mindestens einen GHR-Reformerinnenraum mit darin angeordneten GHR-Reformerrohren, einen GHR-Rohgaseinlass verbunden mit den GHR-Reformerrohren; eine GHR-Ummantelung der GHR-Reformerrohre; einen GHR-Hauptreformerproduktgaseinlass verbunden mit der Hauptreformer-Produkt-Ableitung und der GHR-Ummantelung.

Bevorzugt weist der nicht umgesetzte Nebenstrom eine Temperatur von 250 °C bis 500 °C auf.

Bevorzugt weist der umgesetzte Hauptstrom eine Temperatur von 700 °C bis 1000 °C auf. Die Erfindung umfasst des Weiteren ein Verfahren zur Nachrüstung einer Anlage zur Herstellung von Wasserstoff, Ammoniak, Synthesegas, Harnstoff und/oder Methanol umfassend den Einbau und/oder Umbau der erfindungsgemäßen Reformeranordnung. Weiterhin umfasst die Erfindung die Verwendung der erfindungsgemäßen Reformeranordnung zur Herstellung von Wasserstoff, Ammoniak, Synthesegas, Harnstoff und/oder Methanol.

Die Erfindung umfasst außerdem eine Anlage zur Herstellung von Wasserstoff, Ammoniak, Synthesegas, Harnstoff, Erdölraffinerien, und/oder Methanol umfassend die erfindungsgemäße Reformeranordnung.

Des Weiteren wird die Erfindung anhand der folgenden Figuren näher erläutert. Die Figuren beschränken dabei nicht den Schutzumfang der Erfindung, sondern dienen nur der beispielhaften Erläuterung. Die Figuren sind nicht maßstabsgetreu.

Es zeigen:
Figur 1 einen Querschnitt einer Reformeranordnung zur Herstellung von Synthesegas und/oder Wasserstoff nach dem Stand der Technik,
Figur 2 den Grundaufbau eines Querschnitts einer erfindungsgemäßen Reformeranordnung zur Herstellung von Synthesegas und/oder Wasserstoff und
Figur 3 einen weiteren Querschnitt einer erfindungsgemäßen Reformeranordnung zur Herstellung von Synthesegas und/oder Wasserstoff.

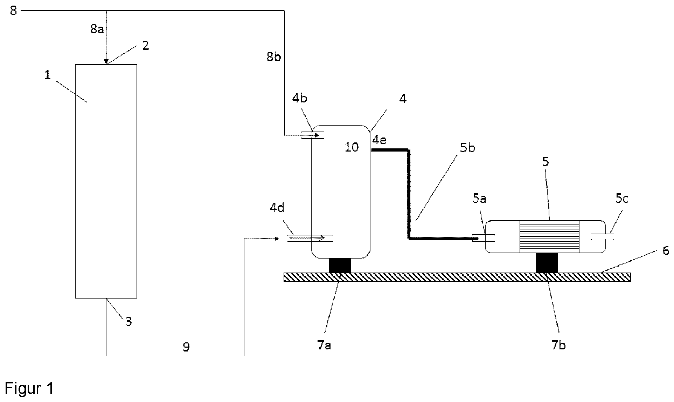

Figur 1 zeigt einen Querschnitt einer Reformeranordnung zur Herstellung von Synthesegas und/oder Wasserstoff nach dem Stand der Technik. Eine Zuleitung (8) stellt ein Kohlenwasserstoff (z.B. Erdgas)/Dampf Gemisch bereit. Die Reformeranordnung zur Herstellung von Synthesegas und/oder Wasserstoff umfasst die folgenden Elemente. Ein Hauptreformer (1), beispielsweise ein Primärreformer, ist mit einer Hauptreformer-Edukt-Zuleitung (2) der abgezweigten Zuleitung (8a) und einer Hauptreformer-Produkt-Ableitung (3, 9) verbunden. Weiterhin ist ein gasbeheizter Reformer (GHR) (4) enthalten. Die Reformeranordnung umfasst weiterhin einen Wärmeüberträger (5) mit einer Wärmeüberträgereinlassöffnung (5a) und einer Wärmeüberträgerauslassöffnung (5c), wobei ein Wärmekanal (5b) zwischen der Wärmeüberträgereinlassöffnung (5a) und dem GHR-Produktauslass (4e) angeordnet ist. Beispiele für geeignete Wärmeüberträger (5) sind Prozessgaskühler und/oder Prozesskondensatverdampfer und/oder Dampfüberhitzer. Der umgesetzte Hauptstrom (9) gelangt aus dem Hauptreformer (1) in den GHR-Hauptreformerproduktgaseinlass (4d). Ein Nebenstrom (8b) gelangt in den gasbeheizten Reformer (GHR/Gas Heated Reformer) (4) über einen GHR-Rohgaseinlass (4b). Weiterhin sind ein GHR-Produktauslass (4e) zum Transport des gemischten Stroms (10) über einen Wärmekanal (5b) mit einer Wärmeüberträgereinlassöffnung (5a) des Wärmeüberträgers (5) verbunden. Der gasbeheizte Reformer (GHR) (4) und der Wärmeüberträger (5) sind über Halterungen (7a/7b) auf einer Bodenplatte (6) angeordnet.

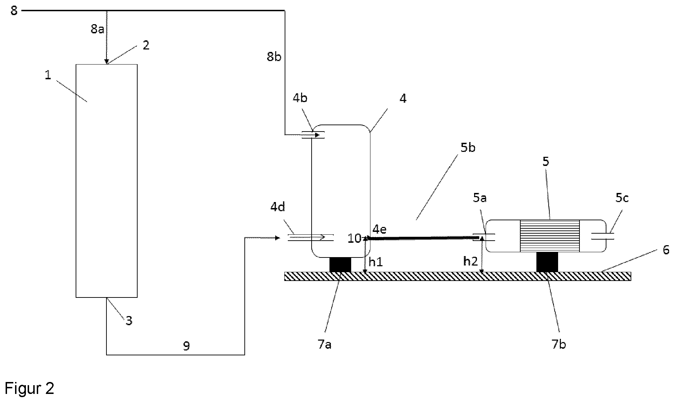

Figur 2 zeigt den Grundaufbau eines Querschnitts einer erfindungsgemäßen Reformeranordnung zur Herstellung von Synthesegas und/oder Wasserstoff. Der grundsätzliche Aufbau bis auf die Anordnung des Wärmekanals (5b), GHR-Produktauslass (4e) und der Wärmeüberträgereinlassöffnung (5a) sind identisch mit dem in Figur 1 gezeigten Aufbau. Der GHR-Produktauslass (4e) ist auf einer parallelen Ebene mit dem Wärmeüberträger (5) angeordnet ist, wobei sich die Höhe (h1) gemessen vom Boden bis zur Mittelachse des GHR-Produktauslass (4e) um maximal plus/minus 20 % von der Höhe (h2) gemessen vom Boden bis zum zur Mittelachse der Wärmeüberträgereinlassöffnung (5a) unterscheidet. Aufgrund dieser erfindungsgemäßen parallelen Anordnung muss nur eine minimale Verbindungsstrecke als Wärmekanal (5b) zwischen dem gasbeheizten Reformer (GHR/Gas Heated Reformer) (4) und dem Wärmeüberträger (5) wärmeisoliert werden.

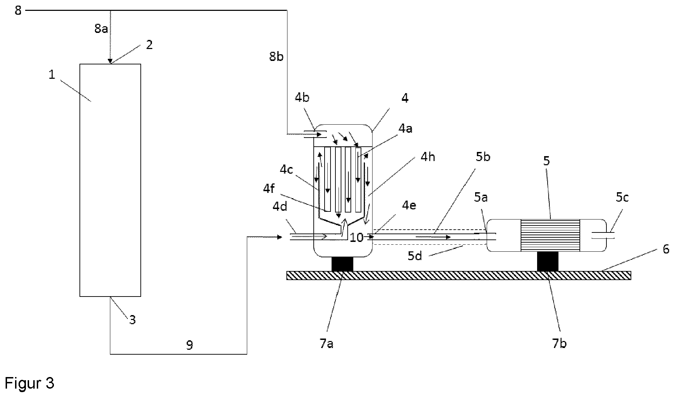

Figur 3 zeigt einen weiteren Querschnitt einer erfindungsgemäßen Reformeranordnung zur Herstellung von Synthesegas und/oder Wasserstoff. Der Grundaufbau entspricht dem in Figur 2 gezeigten Aufbau. Der gasbeheizten Reformer (GHR/Gas Heated Reformer) (4) umfasst mindestens einen GHR-Reformerinnenraum (4h) mit darin angeordneten GHR-Reformerrohren (4a), einen GHR-Rohgaseinlass (4b) verbunden mit den GHR-Reformerrohren (4a); eine GHR-Ummantelung (4c) der GHR-Reformerrohre (4a); einen GHR-Hauptreformerproduktgaseinlass (4d) verbunden mit der Hauptreformer-Produkt-Ableitung und der GHR-Ummantelung (4c). Der Wärmekanal (5b) ist von einer Wärmekanalisolierung (5d) umgeben. Die Pfeile geben die Stromrichtung der Prozessgase an.

### Bezugszeichenliste

- (1): Hauptreformer
- (2): Hauptreformer-Edukt-Zuleitung
- (3): Hauptreformer-Produkt-Ableitung (3)
- (4): gasbeheizter Reformer
- (4a): GHR-Reformerrohre
- (4b): GHR-Rohgaseinlass
- (4c): GHR-Ummantelung
- (4d): GHR-Hauptreformerproduktgaseinlass
- (4e): GHR-Produktauslass
- (4f): Auslässe der GHR-Reformerrohre
- (4h): (Beheizter) Innenraum des GHRs
- (5): Wärmeüberträger
- (5a): Wärmeüberträgereinlassöffnung
- (5b): Wärmekanal
- (5c): Wärmeüberträgerauslassöffnung
- (5d): Wärmekanalisolierung
- (6): Bodenplatte
- (7a): Halterungen
- (7b): Halterungen
- (8): Zuleitung
- (8a): abgezweigten Zuleitung
- (8b): Nebenstrom
- (9): umgesetzte Hauptstrom
- (10): gemischter Strom aus GHR und Hauptreformer

## Patentansprüche

1. Reformeranordnung zur Herstellung von Synthesegas und/oder Wasserstoff mindestens umfassend die folgenden Elemente:
- ein Hauptreformer (1) verbunden mit einer Hauptreformer-Edukt-Zuleitung (2) und einer Hauptreformer-Produkt-Ableitung (3);
- ein gasbeheizter Reformer (GHR/Gas Heated Reformer) (4) umfassend einen GHR-Reformerinnenraum (4h) mit darin angeordneten GHR-Reformerrohren (4a), einen GHR-Rohgaseinlass (4b) verbunden mit den GHR-Reformerrohren (4a); einen GHR-Hauptreformerproduktgaseinlass (4d) verbunden mit der Hauptreformer-Produkt-Ableitung (3); einen GHR-Produktauslass (4e);
- einen Wärmeüberträger (5) mit einer Wärmeüberträgereinlassöffnung (5a) und einer Wärmeüberträgerauslassöffnung (5c), wobei ein Wärmekanal (5b) zwischen der Wärmeüberträgereinlassöffnung (5a) und dem GHR-Produktauslass (4e) angeordnet ist;
**dadurch gekennzeichnet, dass** der gasbeheizte Reformer (4) eine GHR-Ummantelung (4c) der GHR-Reformerrohre (4a) umfasst, die im GHR-Reformerinnenraum (4h) angeordnet ist, wobei der GHR-Hauptreformerprodukteinlass (4d) mit der GHR-Ummantelung (4c) verbunden ist und in diese einmündet, Auslässe (4f) der GHR-Reformerrohre (4a) innerhalb der GHR-Ummantelung (4c) angeordnet sind und die GHR-Ummantelung (4c) eine Öffnung (4g) innerhalb des GHR-Reformerinnenraums (4h) aufweist, und wobei der GHR-Produktauslass (4e) auf einer parallelen Ebene mit dem Wärmeüberträger (5) angeordnet ist und sich die Höhe gemessen vom Boden bis zur Mittelachse des GHR-Produktauslass (4e) um maximal plus/minus 20 % von der Höhe gemessen vom Boden bis zur Mittelachse der Wärmeüberträgereinlassöffnung (5a) unterscheidet.

2. Reformeranordnung nach Anspruch 1, **dadurch gekennzeichnet, dass** der Hauptreformer (1) Primärreformer, Sekundärreformer, POX-Reaktor und/oder ATR-Reformer umfasst.

3. Reformeranordnung nach Anspruch 1 oder 2, **dadurch gekennzeichnet, dass** der Wärmeüberträger Prozessgaskühler und/oder Prozesskondensatverdampfer und/oder Dampfüberhitzer umfasst.

4. Reformeranordnung nach einem der voranstehenden Ansprüche, **dadurch gekennzeichnet, dass** der GHR-Produktauslass (4e) direkt in kürzester, gerader Strecke über den Wärmekanal (5b) mit dem Wärmeüberträger (5) verbunden ist.

5. Reformeranordnung nach einem der voranstehenden Ansprüche, **dadurch gekennzeichnet, dass** zwischen der Hauptreformer-Produkt-Ableitung (3) und dem GHR-Hauptreformerproduktgaseinlass (4d) ein Sekundärreformer angeordnet ist.

6. Reformeranordnung nach einem der voranstehenden Ansprüche, **dadurch gekennzeichnet, dass** der Wärmekanal (5b) von einer Wärmekanalisolierung (5d) umgeben ist.

7. Verfahren zur Reformierung eines Gasgemisches aus Dampf und Kohlenwasserstoffen mindestens umfassend die folgenden Schritte:
a.) Bereitstellen eines Gasgemisches (8) aus Wasserdampf und einen gasförmigen Kohlenwasserstoff, insbesondere Erdgas, Naphtha, LPG, kohlenwasserstoffhaltige Raffineriegase, sowie vorreformiertes Gas;
b.) Einleiten und Umsetzen von 70 Vol. % bis 90 Vol. % des Gasgemisches als Hauptstrom (8a) in einen Hauptreformer (1) und Einleiten von 30 Vol. % bis 10 Vol. % des Gasgemisches als Nebenstrom (8b) in einen gasbeheizten Reformer (GHR/Gas Heated Reformer) (4); wobei ein GHR-Produktauslass (4e) auf einer parallelen Ebene mit einem Wärmeüberträger (5) angeordnet ist und sich die Höhe gemessen vom Boden bis zur Mittelachse des GHR-Produktauslass (4e) um maximal plus/minus 20 % von der Höhe gemessen vom Boden bis zum zur Mittelachse der Wärmeüberträgereinlassöffnung (5a) unterscheidet, wobei der gasbeheizte Reformer (4) mindestens umfasst:
- einen GHR-Reformerinnenraum (4h) mit darin angeordneten GHR-Reformerrohren (4a),
- einen GHR-Rohgaseinlass (4b) verbunden mit den GHR-Reformerrohren (4a);
- eine GHR-Ummantelung (4c) der GHR-Reformerrohre (4a), die im GHR-Reformerinnenraum (4h) angeordnet ist, wobei Auslässe (4f) der GHR-Reformerrohre (4a) innerhalb der GHR-Ummantelung (4c) angeordnet sind und die GHR-Ummantelung (4c) eine Öffnung (4g) innerhalb des GHR-Reformerinnenraums (4h) aufweist;
- einen GHR-Hauptreformerproduktgaseinlass (4d) verbunden mit der Hauptreformer-Produkt-Ableitung (3) und der GHR-Ummantelung (4c), wobei der GHR-Hauptreformerproduktgaseinlass (4d) in die GHR-Ummantelung einmündet;
c.) Einleiten des umgesetzten Hauptstroms (9) aus dem Hauptreformer (1) in den GHR-Hauptreformerproduktgaseinlass (4d);
d.) Erwärmen des Nebenstroms in dem GHR-Reformerinnenraum (4h) über den umgesetzten Hauptstrom und Erhalten eines gemischten Stroms (10) aus umgesetzten Hauptstrom und umgesetzten Nebenstrom;
e.) Einleiten des gemischten Stroms (10) in den Wärmeüberträger (5) über einen Wärmekanal (5b) zwischen der Wärmeüberträgereinlassöffnung (5a) und dem GHR-Produktauslass (4e).

8. Verfahren nach Anspruch 7, **dadurch gekennzeichnet, dass** der Hauptreformer (1) Primärreformer, Sekundärreformer und/oder ATR-Reformer und/oder POX umfasst.

9. Verfahren nach Anspruch 7 oder 8, **dadurch gekennzeichnet, dass** der der nicht umgesetzte Nebenstrom (8b) eine Temperatur von 250 °C bis 500 °C aufweist.

10. Verfahren nach einem der Ansprüchen 7 bis 9, **dadurch gekennzeichnet, dass** der umgesetzte Hauptstrom (9) eine Temperatur von 700 °C bis 1000 °C aufweist.

11. Verfahren zur Nachrüstung einer Anlage zur Herstellung von Wasserstoff, Ammoniak, Synthesegas, Harnstoff und/oder Methanol umfassend den Einbau und/oder Umbau einer Reformeranordnung nach einem der Ansprüche 1 bis 6.

12. Verwendung einer Reformeranordnung nach einem der Ansprüche 1 bis 6 zur Herstellung von Wasserstoff, Ammoniak, Synthesegas, Harnstoff und/oder Methanol.

13. Anlage zur Herstellung von Wasserstoff, Ammoniak, Synthesegas, Harnstoff, Erdölraffinerien und/oder Methanol umfassend eine Reformeranordnung nach einem der Ansprüche 1 bis 6.

## Claims

1. Reformer arrangement for the production of synthesis gas and/or hydrogen at least comprising the following elements:
- a main reformer (1) connected to a main reformer educt feed line (2) and a main reformer product discharge line (3);
- a gas heated reformer (GHR) (4) comprising a GHR reformer interior (4h) having GHR reformer tubes (4a) disposed therein, a GHR raw gas inlet (4b) connected to the GHR reformer tubes (4a); a GHR main reformer product gas inlet (4d) connected to the main reformer product discharge line (3); a GHR product outlet (4e);
- a heat exchanger (5) having a heat exchanger inlet port (5a) and a heat exchanger outlet port (5c), wherein a heat channel (5b) is disposed between the heat exchanger inlet port (5a) and the GHR product outlet (4e);
**characterized in that** the gas heated reformer (4) comprises a GHR shell (4c) of the GHR reformer tubes (4a) disposed in the GHR reformer interior (4h), the GHR main reformer product inlet (4d) being connected to and opening into the GHR shell (4c), outlets (4f) of the GHR reformer tubes (4a) are disposed within the GHR shell (4c), and the GHR shell (4c) has an opening (4g) within the GHR reformer interior (4h), and wherein the GHR product outlet (4e) is arranged on a parallel plane with the heat exchanger (5) and the height measured from the bottom to the center axis of the GHR product outlet (4e) differs by a maximum of plus/minus 20% from the height measured from the bottom to the center axis of the heat exchanger inlet opening (5a).

2. Reformer arrangement according to claim 1, **characterized in that** the main reformer (1) comprises primary reformer, secondary reformer, POX reactor and/or ATR reformer.

3. Reformer arrangement according to claim 1 or 2, **characterized in that** the heat exchanger comprises process gas cooler and/or process condensate evaporator and/or steam superheater.

4. Reformer arrangement according to one of the preceding claims, **characterized in that** the GHR product outlet (4e) is directly connected to the heat exchanger (5) in the shortest straight section via the heat channel (5b).

5. Reformer arrangement according to one of the preceding claims, **characterized in that** a secondary reformer is arranged between the main reformer product discharge line (3) and the GHR main reformer product gas inlet (4d).

6. Reformer arrangement according to any of the preceding claims, **characterized in that** the heat channel (5b) is surrounded by a heat channel insulation (5d).

7. Process for reforming a gas mixture of steam and hydrocarbons at least comprising the following steps:
a.) providing a gas mixture (8) of steam and a gaseous hydrocarbon, in particular natural gas, naphtha, LPG, hydrocarbon-containing refinery gases, and pre-reformed gas;
b.) introducing and converting 70 vol. % to 90 vol. % of the gas mixture as main stream (8a) into a main reformer (1) and introducing 30 vol. % to 10 vol. % of the gas mixture as a side stream (8b) into a gas heated reformer (GHR) (4); wherein a GHR product outlet (4e) is arranged on a parallel plane with a heat exchanger (5) and the height measured from the bottom to the center axis of the GHR product outlet (4e) differs by at most plus/minus 20 % from the height measured from the bottom to the center axis of the heat exchanger inlet (5a), wherein the gas heated reformer (4) comprises at least:
- a GHR reformer interior (4h) having GHR reformer tubes (4a) disposed therein,
- a GHR raw gas inlet (4b) connected to the GHR reformer tubes (4a);
- a GHR shell (4c) of the GHR reformer tubes (4a) disposed in the GHR reformer interior (4h), wherein outlets (4f) of the GHR reformer tubes (4a) are disposed within the GHR shell (4c) and the GHR shell (4c) has an opening (4g) within the GHR interior (4h);
- a GHR main reformer product gas inlet (4d) connected to the main reformer product discharge line (3) and the GHR shell (4c), the GHR main reformer product gas inlet (4d) opening into the GHR shell;
c.) introducing the reacted main stream (9) from the main reformer (1) into the GHR main reformer product gas inlet (4d);
d.) heating the side stream in the GHR reformer interior (4h) over the reacted main stream and obtaining a mixed stream (10) of reacted main stream and reacted side stream;
e.) introducing the mixed stream (10) into the heat exchanger (5) via a heat channel (5b) between the heat exchanger inlet port (5a) and the GHR product outlet (4e).

8. Method according to claim 7, **characterized in that** the main reformer (1) comprises primary reformer, secondary reformer and/or ATR reformer and/or POX.

9. Process according to claim 7 or 8, **characterized in that** the unreacted secondary stream (8b) has a temperature of 250 °C to 500 °C.

10. Process according to any one of claims 7 to 9, **characterized in that** the reacted main stream (9) has a temperature of 700 °C to 1000 °C.

11. Process for retrofitting a plant for the production of hydrogen, ammonia, synthesis gas, urea and/or methanol comprising the installation and/or conversion of a reformer arrangement according to any one of claims 1 to 6.

12. Use of a reformer arrangement according to any one of claims 1 to 6 for the production of hydrogen, ammonia, synthesis gas, urea and/or methanol.

13. Plant for the production of hydrogen, ammonia, synthesis gas, urea, petroleum refineries and/or methanol comprising a reformer arrangement according to any one of claims 1 to 6.

## Revendications

1. Dispositif de reformage pour la production de gaz de synthèse et/ou d'hydrogène comprenant au moins les éléments suivants :
- un reformeur principal (1) relié à une conduite d'alimentation en produit du reformeur principal (2) et à une conduite d'évacuation du produit du reformeur principal (3) ;
- un reformeur chauffé au gaz (GHR/Gas Heated Reformer) (4) comprenant un espace intérieur de reformeur GHR (4h) avec des tubes de reformeur GHR (4a) disposés à l'intérieur, une entrée de gaz brut GHR (4b) reliée aux tubes de reformeur GHR (4a) ; une entrée de gaz de produit de reformeur principal GHR (4d) reliée à la conduite de sortie de produit de reformeur principal (3) ; une sortie de produit GHR (4e) ;
- un échangeur de chaleur (5) ayant une ouverture d'entrée d'échangeur de chaleur (5a) et une ouverture de sortie d'échangeur de chaleur (5c), dans lequel un canal de chaleur (5b) est disposé entre l'ouverture d'entrée d'échangeur de chaleur (5a) et la sortie de produit GHR (4e) ;
**caractérisé en ce que** le reformeur chauffé au gaz (4) comprend une enveloppe de GHR (4c) des tubes de reformeur de GHR (4a) disposée dans l'intérieur du reformeur de GHR (4h), l'entrée de produit de reformeur principal de GHR (4d) étant reliée à l'enveloppe de GHR (4c) et débouchant dans celle-ci, des sorties (4f) des tubes de reformeur GHR (4a) sont disposées à l'intérieur de l'enveloppe GHR (4c) et l'enveloppe GHR (4c) comporte une ouverture (4g) à l'intérieur de l'espace intérieur de reformeur GHR (4h), et dans lequel la sortie de produit GHR (4e) est disposée sur un plan parallèle à l'échangeur de chaleur (5) et la hauteur mesurée du sol à l'axe central de la sortie de produit GHR (4e) diffère au maximum de plus ou moins 20 % de la hauteur mesurée du sol à l'axe central de l'ouverture d'entrée d'échangeur de chaleur (5a).

2. Dispositif de reformage selon la revendication 1, **caractérisé en ce que** le reformeur principal (1) comprend un reformeur primaire, un reformeur secondaire, un réacteur POX et/ou un reformeur ATR.

3. Dispositif de reformage selon la revendication 1 ou 2, **caractérisé en ce que** l'échangeur de chaleur comprend un refroidisseur de gaz de procédé et/ou un évaporateur de condensat de procédé et/ou un surchauffeur de vapeur.

4. Dispositif de reformage selon l'une quelconque des revendications précédentes, **caractérisé en ce que** la sortie de produit GHR (4e) est directement reliée à l'échangeur de chaleur (5) dans la distance droite la plus courte via le canal de chaleur (5b).

5. Dispositif de reformage selon l'une des revendications précédentes, **caractérisé en ce qu'**un reformeur secondaire est disposé entre la dérivation de produit du reformeur principal (3) et l'entrée de gaz de produit de reformeur principal GHR (4d).

6. Dispositif de reformage selon l'une des revendications précédentes, **caractérisé en ce que** le canal de chaleur (5b) est entouré d'une isolation de canal de chaleur (5d).

7. Procédé de reformage d'un mélange gazeux de vapeur et d'hydrocarbures, comprenant au moins les étapes suivantes :
a.) Préparation d'un mélange gazeux (8) de vapeur d'eau et d'un hydrocarbure gazeux, en particulier du gaz naturel, du naphta, du GPL, des gaz de raffinerie contenant des hydrocarbures, ainsi que du gaz préreformé ;
b.) introduction et conversion de 70 % à 90 % en volume du mélange gazeux en tant que courant principal (8a) dans un reformeur principal (1) et introduction de 30 % à 10 % en volume du mélange gazeux en tant que courant secondaire (8b). % du mélange gazeux en tant que flux secondaire (8b) dans un reformeur chauffé au gaz (GHR/Gas Heated Reformer) (4) ; dans lequel une sortie de produit GHR (4e) est disposée sur un plan parallèle avec un échangeur de chaleur (5) et la hauteur mesurée du sol à l'axe central de la sortie de produit GHR (4e) diffère au maximum de plus ou moins 20 % de la hauteur mesurée du sol à l'axe central de l'ouverture d'entrée de l'échangeur de chaleur (5a), dans lequel le reformeur chauffé au gaz (4) comprend au moins :
- un espace intérieur de reformeur GHR (4h) avec des tubes de reformeur GHR (4a) disposés à l'intérieur,
- une entrée de gaz brut GHR (4b) reliée aux tubes de reformeur GHR (4a) ;
- une enveloppe de GHR (4c) des tubes de reformeur GHR (4a) disposée dans l'espace intérieur de reformeur de GHR (4h), des sorties (4f) des tubes de reformeur GHR (4a) étant disposées à l'intérieur de l'enveloppe de GHR (4c), l'enveloppe de GHR (4c) ayant une ouverture (4g) à l'intérieur de l'espace intérieur de reformeur de GHR (4h) ;
- une entrée de gaz de produit de reformeur GHR principal (4d) reliée à la conduite d'évacuation de produit du reformeur principal (3) et à l'enveloppe de GHR (4c), l'entrée de gaz de produit de reformeur GHR principal (4d) débouchant dans l'enveloppe de GHR ;
c.) Introduction du flux principal converti (9) provenant du reformeur principal (1) dans l'entrée de gaz de produit de reformeur principal GHR (4d) ;
d.) Chauffage du courant secondaire dans l'intérieur du reformeur GHR (4h) au-dessus du courant principal ayant réagi et obtention d'un courant mixte (10) de courant principal ayant réagi et de courant secondaire ayant réagi ;
e.) Introduction du flux mélangé (10) dans l'échangeur de chaleur (5) via un canal de chaleur (5b) entre l'orifice d'entrée de l'échangeur de chaleur (5a) et l'orifice de sortie du produit GHR (4e).

8. Procédé selon la revendication 7, **caractérisé en ce que** le reformeur principal (1) comprend des reformeurs primaires, des reformeurs secondaires et/ou des reformeurs ATR et/ou des POX.

9. Procédé selon la revendication 7 ou 8, **caractérisé en ce que** le flux secondaire (8b) non converti présente une température de 250°C à 500°C.

10. Procédé selon l'une des revendications 7 à 9, **caractérisé en ce que** le courant principal (9) ayant réagi présente une température de 700 °C à 1000 °C.

11. Procédé de mise à niveau d'une installation de production d'hydrogène, d'ammoniac, de gaz de synthèse, d'urée et/ou de méthanol, comprenant l'installation et/ou la transformation d'un agencement de reformeur selon l'une des revendications 1 à 6.

12. Utilisation d'un dispositif de reformage selon l'une des revendications 1 à 6 pour la production d'hydrogène, d'ammoniac, de gaz de synthèse, d'urée et/ou de méthanol.

13. Installation pour la production d'hydrogène, d'ammoniac, de gaz de synthèse, d'urée, de pétrole raffiné et/ou de méthanol comprenant un agencement de reformeur selon l'une des revendications 1 à 6.
